# EUROPEAN PATENT APPLICATION

(11) **EP 2 253 319 A1**
(43) Date of publication of application: **24.11.2010**
(21) Application number: 10173158.6
(22) Date of filing: 15.05.2002
(51) Int. Cl.: A61K 31/506, A61P 35/00, A61P 37/06

(54) **Combination comprising N-{5-[4-(4-methyl-piperazino-methyl)-benzoylamido]-2-methylphenyl}-4-(3-pyridyl)-2pyrimidine-amine and a chemotherapeutic agent.**

(30) Priority: 16.05.2001 US 291427 P
(62) Divisional of application: 06011049.1
(71) Applicant: Novartis AG, 4056 Basel (CH); Novartis Pharma GmbH, 1230 Wien (AT)
(72) Inventor: Bruns, Christian, 79110, Freiburg (DE); Buchdunger, Elisabeth, 79395, Neuenburg (DE); O'Reilly, Terence, 4057, Basel (CH); Silberman, Sandra Leta, Randolph, NJ 07869 (US); Wartmann, Markus, 4125, Riehen (CH); Weckbecker, Gisbert, 4105, Biel-Benken (CH)
(74) Representative: Pfister-Fu, Yixin

(57) **Abstract**

A method of treating a warm-blooded animal, especially a human, having a proliferative disease or acute or chronic transplant rejection comprising administering to the animal a combination which comprises (a) N-{5-[4-(4-methyl-piperazino-methyl)-benzoylamido]-2-methylphenyl}-4-(3-pyridyl)-2-pyrimidine-amine and (b) a chemotherapeutic agent selected from antineoplastic agents, especially as defined herein, and agents effective in treating acute or chronic transplant rejection; a combination comprising (a) and (b) as defined above and optionally at least one pharmaceutically acceptable carrier for simultaneous, separate or sequential use, in particular for the delay of progression or treatment of a proliferative disease, especially a solid tumor disease.

## Description

The invention relates to a method of treating a warm-blooded animal, especially a human, having a proliferative disease or acute or chronic transplant rejection comprising administering to the animal a combination which comprises (a) N-{5-[4-(4-methyl-piperazinomethyl)-benzoylamido]-2-methylphenyl}-4-(3-pyridyl)-2-pyrimidine-amine and (b) a chemotherapeutic agent selected from antineoplastic agents, especially as defined herein, and agents effective in treating acute or chronic transplant rejection; a combination comprising (a) and (b) as defined above and optionally at least one pharmaceutically acceptable carrier for simultaneous, separate or sequential use, in particular for the delay of progression or treatment of a proliferative disease, especially a solid tumor disease; a pharmaceutical composition comprising such a combination; the use of such a combination for the preparation of a medicament for the delay of progression or treatment of a proliferative disease; and to a commercial package or product comprising such a combination.

Surprisingly, it has been found that the antineoplastic effect, i.e. especially the delay of progression or treatment of a proliferative disease, in particular the treatment of a tumor that is refractory to other chemotherapeutics known as antineoplastic agents, and/or the effect in treating acute or chronic transplant rejection, of a combination as defined herein is greater than the effects that can be achieved with either type of combination partner alone, i.e. greater than the effects of a monotherapy using only one of the combination partners (a) and (b) as defined herein.

Hence, the present invention relates to a method of treating a warm-blooded animal having a proliferative disease and/or acute or chronic transplant rejection comprising administering to the animal a combination which comprises (a) N-{5-[4-(4-methyl-piperazino-methyl)-benzoylamido]-2-methylphenyl}-4-(3-pyridyl)-2-pyrimidine-amine and (b) a chemotherapeutic agent selected from antineoplastic agents and agents effective in treating acute or chronic transplant rejection, in a quantity which is jointly therapeutically effective against a proliferative disease or acute or chronic transplant rejection and in which the compounds can also be present in the form of their pharmaceutically acceptable salts.

Furthermore, the present invention pertains to a combination, such as a combined preparation or a pharmaceutical composition, which comprises (a) N-{5-[4-(4-methyl-piperazino-methyl)-benzoylamido]-2-methylphenyl}-4-(3-pyridyl)-2-pyrimidine-amine and (b) a chemotherapeutic agent selected from antineoplastic agents and agents effective in treating acute or chronic transplant rejection, wherein the active ingredients are present in each case in free form or in the form of a pharmaceutically acceptable salt, and optionally at least one pharmaceutically acceptable carrier; for simultaneous, separate or sequential use.

The term "a combined preparation", as used herein defines especially a "kit of parts" in the sense that the combination partners (a) and (b) as defined above can be dosed independently or by use of different fixed combinations with distinguished amounts of the combination partners (a) and (b), i.e., simultaneously or at different time points. The parts of the kit of parts can then, e.g., be administered simultaneously or chronologically staggered, that is at different time points and with equal or different time intervals for any part of the kit of parts. Very preferably, the time intervals are chosen such that the effect on the treated disease in the combined use of the parts is larger than the effect which would be obtained by use of only any one of the combination partners (a) and (b). The ratio of the total amounts of the combination partner (a) to the combination partner (b) to be administered in the combined preparation can be varied, e.g. in order to cope with the needs of a patient sub-population to be treated or the needs of the single patient which different needs can be due to the particular disease, age, sex, body weight, etc. of the patients. Preferably, there is at least one beneficial effect, e.g., a mutual enhancing of the effect of the combination partners (a) and (b), in particular a synergism, e.g. a more than additive effect, additional advantageous effects, less side effects, a combined therapeutical effect in a non-effective dosage of one or both of the combination partners (a) and (b), and very preferably a strong synergism of the combination partners (a) and (b).

The term "delay of progression" as used herein means administration of the combination to patients being in a pre-stage or in an early phase of the disease to be treated, in which patients for example a pre-form of the corresponding disease is diagnosed or which patients are in a condition, e.g. during a medical treatment or a condition resulting from an accident, under which it is likely that a corresponding disease will develop.

The term "solid tumor" especially means breast cancer, cancer of the colon and generally the GI tract, lung cancer, in particular small-cell lung cancer, and non-small-cell lung cancer, head and neck cancer, genitourinary cancer, e.g. cervical, uterine, ovarian, testicles, prostate or bladder cancer; Hodgkin's disease or Kaposi's sarcoma. The combinations of the present invention inhibit the growth of liquid tumors and, in particular, solid tumors. Furthermore, depending on the tumor type and the particular combination used a decrease of the tumor volume can be obtained. The combinations disclosed herein are also suited to prevent the metastatic spread of tumors and the growth or development of micrometastases. The combinations disclosed herein are in particular suitable for the treatment of poor prognosis patients, e.g. such poor prognosis patients having non-small-cell lung cancer.

It will be understood that references to the combination partners (a) and (b) are meant to also include the pharmaceutically acceptable salts. If these combination partners (a) and (b) have, for example, at least one basic center, they can form acid addition salts. Corresponding acid addition salts can also be formed having, if desired, an additionally present basic center. The combination partners (a) and (b) having an acid group (for example COOH) can also form salts with bases. The combination partner (a) or (b) or a pharmaceutically acceptable salt thereof may also be used in form of a hydrate or include other solvents used for crystallization. N-{5-[4-(4-methyl-piperazino-methyl)-benzoylamido]-2-methylphenyl}-4-(3-pyridyl)-2-pyrimidine-amine, i.e. combination partner (a), is preferably used in the present invention in the form of its monomesylate salt (STI571).

The combination partner (a) can be prepared and administered as described in WO 99/03854, especially the monomesylate salt of N-{5-[4-(4-methyl-piperazino-methyl)-benzoylamido]-2-methylphenyl}-4-(3-pyridyl)-2-pyrimidine-amine can be formulated as described in Examples 4 and 6 of WO 99/03854.

The term "antineoplastic agents" as used herein includes, but is not limited to aromatase inhibitors, antiestrogens, topoisomerase I inhibitors, topoisomerase II inhibitors, microtubule active agents, alkylating agents, antineoplastic antimetabolites, platin compounds, compounds decreasing the protein kinase activity and further anti-angiogenic compounds, gonadorelin agonists, anti-androgens, bisphosphonates and trastuzumab.

The term "aromatase inhibitors" as used herein relates to compounds which inhibit the estrogen production, i.e. the conversion of the substrates androstenedione and testosterone to estrone and estradiol, respectively. The term includes, but is not limited to steroids, especially exemestane and formestane and, in particular, non-steroids, especially aminoglutethimide, vorozole, fadrozole, anastrozole and, very especially, letrozole. Exemestane can be administered, e.g., in the form as it is marketed, e.g. under the trademark AROMASIN™. Formestane can be administered, e.g., in the form as it is marketed, e.g. under the trademark LENTARON™. Fadrozole can be administered, e.g., in the form as it is marketed, e.g. under the trademark AFEMA™. Anastrozole can be administered, e.g., in the form as it is marketed, e.g. under the trademark ARIMIDEX™. Letrozole can be administered, e.g., in the form as it is marketed, e.g. under the trademark FEMARA™ or FEMAR™. Letrozole has been specifically described in the European patent No. 0 236 940 published on September 16, 1987, as well as in US patent No. 4,978,672 published on December 18, 1990, and Japanese Patent No. 2018112 all in the name of the applicant. Aminoglutethimide can be administered, e.g., in the form as it is marketed, e.g. under the trademark ORIMETEN™.

A combination of the invention comprising an antineoplastic agent which is an aromatase inhibitor is particularly useful for the treatment of hormone receptor positive breast tumors.

The term "antiestrogens" as used herein relates to compounds which antagonize the effect of estrogens at the estrogen receptor level. The term includes, but is not limited to tamoxifen, fulvestrant, raloxifene and raloxifene hydrochloride. Tamoxifen can be administered, e.g., in the form as it is marketed, e.g. under the trademark NOLVADEX™. Raloxifene hydrochloride can be administered, e.g., in the form as it is marketed, e.g. under the trademark EVISTA™. Fulvestrant can be formulated as disclosed in US 4,659,516 or it can be administered, e.g., in the form as it is marketed, e.g. under the trademark FASLODEX™.

The term "topoisomerase I inhibitors" as used herein includes, but is not limited to topotecan, irinotecan, 9-nitrocamptothecin and the macromolecular camptothecin conjugate PNU-166148 (compound A1 in WO99/17804). Irinotecan can be administered, e.g., in the form as it is marketed, e.g. under the trademark CAMPTOSAR™. Topotecan can be administered, e.g., in the form as it is marketed, e.g. under the trademark HYCAMTIN™. The term "topoisomerase II inhibitors" as used herein includes, but is not limited to the antracyclines doxorubicin (including liposomal formulation, e.g. CAELYX™), epirubicin, idarubicin and nemorubicin, the anthraquinones mitoxantrone and losoxantrone, and the podophillotoxines etoposide and teniposide. Etoposide can be administered, e.g., in the form as it is marketed, e.g. under the trademark ETOPOPHOS™. Teniposide can be administered, e.g., in the form as it is marketed, e.g. under the trademark VM 26-BRISTOL ™. Doxorubicin can be administered, e.g., in the form as it is marketed, e.g. under the trademark ADRIBLASTIN™. Epirubicin can be administered, e.g., in the form as it is marketed, e.g. under the trademark FARMORUBICIN™. Idarubicin can be administered, e.g., in the form as it is marketed, e.g. under the trademark ZAVEDOS™. Mitoxantrone can be administered, e.g., in the form as it is marketed, e.g. under the trademark NOVANTRON™.

The term "microtubule active agents" relates to microtubule stabilizing and microtubule destabilizing agents including, but not limited to the taxanes paclitaxel and docetaxel, the vinca alkaloids, e.g., vinblastine, especially vinblastine sulfate, vincristine especially vincristine sulfate, and vinorelbine, discodermolide and epothilones. Paclitaxel (Taxol^{®)} is a natural product with antitumor activity. TAXOL is obtained via a semi-synthetic process from *Taxus baccata .* The chemical name for paclitaxel is 5(beta),20-Epoxy-1,2(alpha),4,7(beta),10(beta),13(alpha)-hexahydroxytax-11-en-9-one 4,10-diacetate 2-benzoate 13-ester with (2 R ,3 S )- N -benzoyl-3-phenylisoserine. Docetaxel can be administered, e.g., in the form as it is marketed, e.g. under the trademark TAXOTERE™. Vinblastine sulfate can be administered, e.g., in the form as it is marketed, e.g. under the trademark VINBLASTIN R.P.™. Vincristine sulfate can be administered, e.g., in the form as it is marketed, e.g. under the trademark FARMISTIN™. Discodermolide can be obtained, e.g., as disclosed in US 5,010,099.

The term "alkylating agents" as used herein includes, but is not limited to cyclophosphamide, ifosfamide and melphalan. Cyclophosphamide can be administered, e.g., in the form as it is marketed, e.g. under the trademark CYCLOSTIN™. Ifosfamide can be administered, e.g., in the form as it is marketed, e.g. under the trademark HOLOXAN™. The term "antineoplastic antimetabolites" includes, but is not limited to 5-fluorouracil, capecitabine, gemcitabine, methotrexate and edatrexate. Capecitabine can be administered, e.g., in the form as it is marketed, e.g. under the trademark XELODA™. Gemcitabine can be administered, e.g., in the form as it is marketed, e.g. under the trademark GEMZAR™.

The term "platin compounds" as used herein includes, but is not limited to carboplatin, cisplatin and oxaliplatin. Carboplatin can be administered, e.g., in the form as it is marketed, e.g. under the trademark CARBOPLAT™. Oxaliplatin can be administered, e.g., in the form as it is marketed, e.g. under the trademark ELOXATIN™.

The term "compounds decreasing the protein kinase activity and further anti-angiogenic compounds" as used herein includes, but is not limited to compounds decreasing the activity of the epidermal growth factor (EGF) of the epidermal growth factor (EGF), the vascular endothelial growth factor (VEGF), the platelet derived growth factor (PDGF) and/or the protein kinase C and anti-angiogenic compounds having another mechanism for their activity.

Compounds which decreases the activity of VEGF are especially compounds which inhibit the VEGF receptor tyrosine kinase, compounds which inhibit a VEGF receptor and compounds binding to VEGF, and are in particular those compounds, proteins and monoclonal antibodies generically and specifically disclosed in WO 98/35958, WO 00/09495, WO 00/27820, WO 00/59509, WO 98/11223, WO 00/27819 and EP 0 769 947; those as described by M. Prewett et al in Cancer Research 59 (1999) 5209-5218, by F. Yuan et al in Proc. Natl. Acad. Sci. USA, vol. 93, pp. 14765-14770, Dec. 1996, by Z. Zhu et al in Cancer Res. 58, 1998, 3209-3214, and by J. Mordenti et al in Toxicologic Pathology, Vol. 27, no. 1, pp 14-21, 1999; in WO 00/37502 and WO 94/10202; Angiostatin™, described by M. S. O'Reilly et al, Cell 79, 1994, 315-328; and Endostatin™, described by M. S. O'Reilly et al, Cell 88, 1997, 277-285;
compounds which decrease the activity of the epidermal growth factor (EGF) are especially compounds which inhibit the EGF receptor tyrosine kinase, compounds which inhibit the EGF receptor and compounds binding to EGF, and are in particular those compounds generically and specifically disclosed in WO 97/02266, EP 0 564 409, WO 99/03854, EP 0520722, EP 0 566 226, EP 0 787 722, EP 0 837 063, US 5,747,498, WO 98/10767, WO 97/30034, WO 97/49688, WO 97/38983 and, especially, WO 96/33980;
compounds which decreases the activity of the protein kinase C are especially those staurosporine derivatives disclosed in EP 0 296 110 (pharmaceutical preparation described in WO 00/48571) which compounds are protein kinase C inhibitors;
in each case where citations of patent applications or scientific publications are given in particular in the compound claims and the final products of the working examples, the subject-matter of the final products, the pharmaceutical preparations and the claims is hereby incorporated into the present application by reference to this publications. Comprised are likewise the corresponding stereoisomers as well as the corresponding crystal modifications, e.g. solvates and polymorphs, which are disclosed therein. The compounds used as active ingredients in the combinations disclosed herein can be prepared and administered as described in the cited documents, respectively. Further anti-angiogenic compounds are thalidomide (THALOMID), SU5416, and celecoxib (Celebrex);

The term "gonadorelin agonist" as used herein includes, but is not limited to abarelix, goserelin and goserelin acetate. Goserelin is disclosed in US 4,100,274 and can be administered, e.g., in the form as it is marketed, e.g. under the trademark ZOLADEX™. Abarelix can be formulated, eg. as disclosed in US 5,843,901.

The term "anti-androgens" as used herein includes, but is not limited to bicalutamide (CASODEX™), which can be formulated, e.g. as disclosed in US 4,636,505.

The term "bisphosphonates" as used herein includes, but is not limited to etridonic acid, clodronic acid, tiludronic acid, pamidronic acid, alendronic acid, ibandronic acid, risedronic acid and zoledronic acid. "Etridonic acid" can be administered, e.g., in the form as it is marketed, e.g. under the trademark DIDRONEL™. "Clodronic acid" can be administered, e.g., in the form as it is marketed, e.g. under the trademark BONEFOS™. "Tiludronic acid" can be administered, e.g., in the form as it is marketed, e.g. under the trademark SKELID™. "Pamidronic acid" can be administered, e.g., in the form as it is marketed, e.g. under the trademark AREDIA™. "Alendronic acid" can be administered, e.g., in the form as it is marketed, e.g. under the trademark FOSAMAX™. "lbandronic acid" can be administered, e.g., in the form as it is marketed, e.g. under the trademark BONDRANAT™. "Risedronic acid" can be administered, e.g., in the form as it is marketed, e.g. under the trademark ACTONEL™. "Zoledronic acid" can be administered, e.g., in the form as it is marketed, e.g. under the trademark ZOMETA™. Zoledronic acid has been specifically described in the European patent No. 0 275 821, published on July 27, 1988, as well as in US patent No. 4,939,130 granted on July 03, 1990, and Japanese Patent No. 2744238 all in the name of the applicant. The term "Zoledronic acid" also includes pharmacologically acceptable salt thereof or any hydrate thereof such as anhydrous zoledronic acid, zoledronic acid monohydrate, disodium zeldronate, trisodium zeldronate.

"Trastuzumab" can be administered, e.g., in the form as it is marketed, e.g. under the trademark HERCEPTIN™.

The term "agents effective in treating acute or chronic transplant rejection" as used herein relates to a macrocyclic lactone having immunosuppressive properties, e.g. rapamycin, 40-O-(2-hydroxyethyl)-rapamycin (RAD), CCI779 or ABT578; an ascomycin having immunosuppressive properties, e.g. ABT-281, ASM981, etc.; azathioprine; leflunomide; mizoribine; mycophenolic acid; mycophenolate mofetil; a somatostatin analogue like octreotide, lanreotide, vapreotide or SOM230, a calcineurin inhibitor, e.g. cyclosporin A or FK 506; a deoxyspergualine compound or derivative or analog thereof, e.g. 15-DSG, an accelerated lymphocyte homing agent, e.g. 2-amino-2-[2-(4-octylphenyl)ethyl]propane-1,3-diol, FTY720 or a pharmaceutically acceptable salt thereof, preferably a hydrochloride salt therof, corticosteroids, e.g., prednisolone, methylprednisolone and dexamethasone, monoclonal antibodies to leukocyte receptors, e.g., MHC, CD2, CD3, CD4, CD7, CD8, CD11a/CD18, CD25, CD27, CD28, CD40. CD45, CD58, CD80, CD86, CD137, ICOS, CD150 (SLAM), OX40, 4-1 BB or to their ligands, e.g. CD154, or antagonists thereof; other immunomodulatory compounds, e.g. a recombinant binding molecule having at least a portion of the extracellular domain of CTLA4 or a mutant thereof, e.g. an at least extracellular portion of CTLA4 or a mutant thereof joined to a non-CTLA4 protein sequence, e.g. CTLA4Ig (for ex. designated ATCC 68629) or a homologue or a mutant thereof, e.g. LEA29Y ; adhesion molecule inhibitors, e.g. LFA-1 antagonists, ICAM-1 or -3 antagonists, anti-LFA-1 or anti-ICAM antibodies, VCAM-4 antagonists or VLA-4 antagonists; or antichemokine antibodies or antichemokine receptor antibodies or low molecular weight chemokine receptor antagonists, e.g. anti MCP-1 antibodies.

The structure of the active agents identified by code nos., generic or trade names may be taken from the actual edition of the standard compendium "The Merck Index" or from databases, e.g. Patents International (e.g. IMS World Publications). The corresponding content thereof is hereby incorporated by reference.

A combination which comprises (a) N-{5-[4-(4-methyl-piperazino-methyl)-benzoylamido]-2-methylphenyl}-4-(3-pyridyl)-2-pyrimidine-amine and (b) a chemotherapeutic agent selected from antineoplastic agents and agents effective in treating acute or chronic transplant rejection, in which the active ingredients are present in each case in free form or in the form of a pharmaceutically acceptable salt and optionally at least one pharmaceutically acceptable carrier, will be referred to hereinafter as a COMBINATION OF THE INVENTION.

The nature of proliferative diseases like solid tumor diseases and acute or chronic transplant rejection is multifactorial. Under certain circumstances, drugs with different mechanisms of action may be combined. However, just considering any combination of drugs having different mode of action does not necessarily lead to combinations with advantageous effects.

All the more surprising is the experimental finding that *in vivo* the administration of a COMBINATION OF THE INVENTION, especially comprising an antineoplastic agent as combination partner (b), results not only in a beneficial effect, especially a synergistic therapeutic effect, e.g. with regard to slowing down, arresting or reversing the neoplasm formation or a longer duration of tumor response, but also in further surprising beneficial effects, e.g. less side-effects, an improved quality of life and a decreased mortality and morbidity, compared to a monotherapy applying only one of the pharmaceutically active ingredients used in the COMBINATION OF THE INVENTION, in particular in the treatment of a tumor that is refractory to other chemotherapeutics known as anti-cancer agents. In particular, an increased up-take of the combination partner (b) in tumor tissue and tumor cells is observed, when applied in combination with combination partner (a).

A further benefit is that lower doses of the active ingredients of the COMBINATION OF THE INVENTION can be used, for example, that the dosages need not only often be smaller but are also applied less frequently, or can be used in order to diminish the incidence of side-effects. This is in accordance with the desires and requirements of the patients to be treated.

It can be shown by established test models and in particular those test models described herein that a COMBINATION OF THE INVENTION results in a more effective delay of progression or treatment of a proliferative disease and/or acute or chronic transplant rejection compared to the effects observed with the single combination partners. The person skilled in the pertinent art is fully enabled to select a relevant test model to prove the hereinbefore and hereinafter mentioned therapeutic indications and beneficial effects. The pharmacological activity of a COMBINATION OF THE INVENTION may, for example, be demonstrated in a clinical study or in a test procedure as essentially described hereinafter.

Suitable clinical studies are, for example, open label non-randomized, dose escalation studies in patients with advanced solid tumors. Such studies prove in particular the synergism of the active ingredients of the COMBINATIONS OF THE INVENTION. The beneficial effects on proliferative diseases can be determined directly through the results of these studies or by changes in the study design which are known as such to a person skilled in the art. Such studies are, in particular, suitable to compare the effects of a monotherapy using the active ingredients and a COMBINATION OF THE INVENTION. Preferably, the combination partner (a) is administered with a fixed dose and the dose of the combination partner (b) is escalated until the Maximum Tolerated Dosage is reached. In a preferred embodiment of the study, each patient receives daily doses of the combination partner (a). The efficacy of the treatment can be determined in such studies, e.g., after 18 or 24 weeks by radiologic evaluation of the tumors every 6 weeks.

Alternatively, a placebo-controlled, double blind study can be used in order to prove the benefits of the COMBINATION OF THE INVENTION mentioned herein.

The COMBINATION OF THE INVENTION can also be applied in combination with surgical intervention, mild prolonged whole body hyperthermia and/or irradiation therapy.

In a preferred embodiment of the invention the chemotherapeutic agent is an antineoplastic agent selected from aromatase inhibitors, antiestrogens, topoisomerase I inhibitors, topoisomerase II inhibitors, microtubule active agents, alkylating agents, antineoplastic antimetabolites, platin compounds, compounds decreasing the protein kinase activity and further anti-angiogenic compounds, gonadorelin agonists, anti-androgens, bisphosphonates and trastuzumab. In such embodiment the antineoplastic agent is especially selected from pamidronic acid, zoledronic acid and letrozole, preferably zoledronic acid and letrozole.

One embodiment of the invention relates to the use of a COMBINATION OF THE INVENTION for the prevention, delay of progression or treatment of or for the preparation of a medicament for the prevention, delay of progression or treatment of breast cancer. Preferably, in such embodiment the COMBINATION OF THE INVENTION comprises an aromatase inhibitor, very especially the aromatase inhibitor letrozole.

Another embodiment of the invention relates to the use of a COMBINATION OF THE INVENTION for the prevention, delay of progression or treatment of or for the preparation of a medicament for the prevention, delay of progression or treatment of lung cancer. Preferably, in such embodiment the COMBINATION OF THE INVENTION comprises carboplatin.

COMBINATION OF THE INVENTION is also useful for preventing or combating graft vessel diseases, e.g. allo- or xenotransplant vasculopathies, e.g. graft vessel atherosclerosis or chronic graft rejection, e.g. in a transplant of organ, tissue or cells, e.g. heart, lung, combined heart-lung, liver, kidney or pancreatic transplants (e.g. pancreatic islet cells), or for preventing or treating vein graft stenosis, restenosis and/or vascular occlusion following vascular injury, e.g. caused by catherization procedures or vascular scraping procedures such as percutaneous transluminal angioplasty, laser treatment or other invasive procedures which disrupt the integrity of the vascular intima or endothelium.

The COMBINATION OF THE INVENTION can be a combined preparation or a pharmaceutical composition.

It is one objective of this invention to provide a pharmaceutical composition comprising a quantity, which is jointly therapeutically effective against a proliferative disease or acute or chronic transplant rejection comprising the COMBINATION OF THE INVENTION. In this composition, the combination partners (a) and (b) can be administered together, one after the other or separately in one combined unit dosage form or in two separate unit dosage forms. The unit dosage form may also be a fixed combination.

The pharmaceutical compositions for separate administration of the combination partners (a) and (b) and for the administration in a fixed combination, i.e. a single galenical compositions comprising at least two combination partners (a) and (b), according to the invention can be prepared in a manner known per se and are those suitable for enteral, such as oral or rectal, and parenteral administration to mammals (warm-blooded animals), including man, comprising a therapeutically effective amount of at least one pharmacologically active combination partner alone or in combination with one or more pharmaceutically acceptable carries, especially suitable for enteral or parenteral application.

Novel pharmaceutical composition contain, for example, from about 10 % to about 100 %, preferably from about 20 % to about 60 %, of the active ingredients. Pharmaceutical preparations for the combination therapy for enteral or parenteral administration are, for example, those in unit dosage forms, such as sugar-coated tablets, tablets, capsules or suppositories, and furthermore ampoules. If not indicated otherwise, these are prepared in a manner known per se, for example by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can be reached by administration of a plurality of dosage units.

In particular, a therapeutically effective amount of each of the combination partner of the COMBINATION OF THE INVENTION may be administered simultaneously or sequentially and in any order, and the components may be administered separately or as a fixed combination. For example, the method of delay of progression or treatment of a proliferative disease and/or acute or chronic transplant rejection according to the invention may comprise (i) administration of the combination partner (a) in free or pharmaceutically acceptable salt form and (ii) adminstration of a combination partner (b) in free or pharmaceutically acceptable salt form, simultaneously or sequentially in any order, in jointly therapeutically effective amounts, preferably in synergistically effective amounts, e.g. in daily dosages corresponding to the amounts described herein. The individual combination partners of the COMBINATION OF THE INVENTION can be administered separately at different times during the course of therapy or concurrently in divided or single combination forms. Furthermore, the term administering also encompasses the use of a pro-drug of a combination partner that convert *in vivo* to the combination partner as such. The instant invention is therefore to be understood as embracing all such regimes of simultaneous or alternating treatment and the term "administering" is to be interpreted accordingly.

The effective dosage of each of the combination partners employed in the COMBINATION OF THE INVENTION may vary depending on the particular compound or pharmaceutical composition employed, the mode of administration, the condition being treated, the severity of the condition being treated. Thus, the dosage regimen the COMBINATION OF THE INVENTION is selected in accordance with a variety of factors including the route of administration and the renal and hepatic function of the patient. A physician, clinician or veterinarian of ordinary skill can readily determine and prescribe the effective amount of the single active ingredients required to prevent, counter or arrest the progress of the condition. Optimal precision in achieving concentration of the active ingredients within the range that yields efficacy without toxicity requires a regimen based on the kinetics of the active ingredients' availability to target sites.

N-{5-[4-(4-methyl-piperazino-methyl)-benzoylamido]-2-methylphenyl}-4-(3-pyridyl)-2-pyrimidine-amine monomesylate, is preferably administered to a human in a dosage in the range of about 2.5 to 850 mg/day, more preferably 5 to 600 mg/day and most preferably 20 to 300 mg/day. Unless stated otherwise herein, the compound is preferably administered from one to four times per day.

Fadrozole may be administered orally to a human in a dosage range varying from about 0.5 to about 10 mg/day, preferably from about 1 to about 2.5 mg/day.

Letrozole may be administered orally to a human in a dosage range varying from about 0.1 to about 10 mg/day, preferably from about 0.5 to about 5 mg/day, most preferably from about 1 to about 2.5 mg/day. Examples of compositions containing letrozole are well known in the art, e.g. Physicians' Desk Reference Copyright © 2001 (Medical Economics Company Inc).

Single doses of Zoledronic acid should preferably not exceed 8 mg; preferably 4 mg more preferably from about 0.5 to about 4 mg. The duration of an intravenous infusion should preferably be no less than 15 minutes. Compositions containing zoledronic acid are described in the art, e.g. in the International patent application US01/14886 filed on May 5, 2001, and published on November 29, 2001.

Exemestane may be administered orally to a human in a dosage range varying from about 5 to about 200 mg/day, preferably from about 10 to about 25 mg/day, or parenterally from about 50 to 500 mg/day, preferably from about 100 to about 250 mg/day. If the drug shall be administered in a separate pharmaceutical composition, it can be administered in the form disclosed in GB 2,177,700.

Formestane may be administered parenterally to a human in a dosage range varying from about 100 to 500 mg/day, preferably from about 250 to about 300 mg/day.

Anastrozole may be administered orally to a human in a dosage range varying from about 0.25 to 20 mg/day, preferably from about 0.5 to about 2.5 mg/day.

Aminogluthemide may be administered to a human in a dosage range varying from about 200 to 500 mg/day.

Tamoxifen citrate may be administered to a human in a dosage range varying from about 10 to 40 mg/day.

Vinblastine may be administered to a human in a dosage range varying from about 1.5 to 10 mg/m²day.

Vincristine sulfate may be administered parenterally to a human in a dosage range varying from about 0.025 to 0.05 mg/kg body weight * week.

Vinorelbine may be administered to a human in a dosage range varying from about 10 to 50 mg/m²day.

Etoposide phosphate may be administered to a human in a dosage range varying from about 25 to 115 mg/m²day, e.g. 56.8 or 113.6 mg/m²day.

Teniposide may be administered to a human in a dosage range varying from about 75 to 150 mg about every two weeks.

Doxorubicin may be administered to a human in a dosage range varying from about 10 to 100 mg/m²day, e.g. 25 or 50 mg/m²day.

Epirubicin may be administered to a human in a dosage range varying from about 10 to 200 mg/m²day.

Idarubicin may be administered to a human in a dosage range varying from about 0.5 to 50 mg/m²day.

Mitoxantrone may be administered to a human in a dosage range varying from about 2.5 to 25 mg/m²day.

Paclitaxel may be administered to a human in a dosage range varying from about 50 to 300 mg/m²day.

Docetaxel may be administered to a human in a dosage range varying from about 25 to 100 mg/m²day.

Cyclophosphamide may be administered to a human in a dosage range varying from about 50 to 1500 mg/m²day.

Melphalan may be administered to a human in a dosage range varying from about 0.5 to 10 mg/m²day.

5-Fluorouracil may be administered to a human in a dosage range varying from about 50 to 1000 mg/m²day, e.g. 500 mg/m²day.

Capecitabine may be administered to a human in a dosage range varying from about 10 to 1000 mg/m²day.

Gemcitabine hydrochloride may be administered to a human in a dosage range varying from about 1000 mg/week.

Methotrexate may be administered to a human in a dosage range varying from about 5 to 500 mg/m²day.

Topotecan may be administered to a human in a dosage range varying from about 1 to 5 mg/m²day.

Irinotecan may be administered to a human in a dosage range varying from about 50 to 350 mg/m²day.

Carboplatin may be administered to a human in a dosage range varying from about 200 to 400 mg/m² about every four weeks.

Cisplatin may be administered to a human in a dosage range varying from about 25 to 75 mg/m² about every three weeks.

Oxaliplatin may be administered to a human in a dosage range varying from about 50 to 85 mg/m² every two weeks.

Alendronic acid may be administered to a human in a dosage range varying from about 5 to 10 mg/day.

Clodronic acid may be administered to a human e.g. in a dosage range varying from about 750 to 1500 mg/day.

Etridonic acid may be administered to a human in a dosage range varying from about 200 to 400 mg/day.

Ibandronic acid may be administered to a human in a dosage range varying from about 1 to 4 mg every three to four weeks.

Risedronic acid may be administered to a human in a dosage range varying from about 20 to 30 mg/day.

Pamidronic acid may be administered to a human in a dosage range varying from about 15 to 90 mg every three to four weeks.

Tiludronic acid may be administered to a human in a dosage range varying from about 200 to 400 mg/day.

Trastuzumab may be administered to a human in a dosage range varying from about 1 to 4 mg/m²week.

Bicalutamide may be administered to a human in a dosage range varying from about 25 to 50 mg/m²day.

Paclitaxel may be administered to a human in a dosage range varying from about 135 mg/m² to about 175 mg/m² administered intravenously every 3 weeks, or 100 mg/m ² every 2 weeks.

The succinate salt described in Example 62 of WO 98/35958 may be administered to a human in a dosage range of about 50 to 1500, more preferably about 100 to 750, and most preferably 250 to 500, mg/day.

COMBINATION OF THE INVENTION which comprises (a) N-{5-[4-(4-methyl-piperazinomethyl)-benzoylamido]-2-methylphenyl}-4-(3-pyridyl)-2-pyrimidine-amine in a dosage range of about 50 to 500 mg/day, preferably 50 to 200 mg/day and (b) letrozole in a dosage range of about 0.5 to about 5 mg/day preferably about 1 to about 2.5 mg/day.

Furthermore, the present invention pertains to the use of a COMBINATION OF THE INVENTION for the delay of progression or treatment of a proliferative disease and/or acute or chronic transplant rejection and for the preparation of a medicament for the delay of progression or treatment of a proliferative disease and/or acute or chronic transplant rejection.

Moreover, the present invention provides a commercial package comprising as active ingredients COMBINATION OF THE INVENTION, together with instructions for simultaneous, separate or sequential use thereof in the delay of progression or treatment of a proliferative disease and/or acute or chronic transplant rejection.

The following Example illustrates the invention described above, but is not, however, intended to limit the scope of the invention in any way. Other test models known as such to the person skilled in the pertinent art can also determine the beneficial effects of the

### COMBINATION OF THE INVENTION.

### Example 1: DU145 prostate carcinoma human cell lines grown i.d. in nude mice

DU145 prostate carcinoma human cell lines are grown i.d. in nude mice. Tumor cell (10⁶) are injected intradermally (i.d.) on the left and right flank of nude mice. Treatment with compounds is started after 25-32 days when tumors reach a size of 80-100 mm². At this time animals are sorted into groups with equivalent mean and range of tumor sizes. Treatment is then randomized to the different groups. Tumor size is measured with calipers on a weekly basis.

### Example 2: ST1571 alone, Taxol^{®} alone (paclitaxel), doxorubicin alone or the combination of STI571 plus one of both versus rat C6 glioma tumor xenografts in female BALB/c mice

Tumors are initiated by the s.c. injection of 1 x 10⁶ rat C6 cells (n=8/group). When the tumors reaches - 75 mm³, STI571 treatment is begun at 100 mg/kg, p.o., every 12h. The combination partner Taxol^{®} is administered at 15 mg/kg, i.v., every 48 hours, for five times, or the combination partner doxorubicin at 9 mg/kg, i.v., every 7 days.
Data points just spanning the IC50 of the agents alone or in combination are entered into the CalcuSyn program (CalcuSyn, Biosoft, Cambridge UK). This program calculates a non-exclusive combination index (CI), whose value is indicative of the interaction of the two compounds, where CI ∼ 1 represents nearly additive effects; 0.85 - 0.9 indicates a slight synergism and a value below 0.85 indicates synergy.
A C.I. of 0.3 ± 0.03 was obtained for the combination of STI571 and Taxol^{®} and a C.I. of 0.4 ± 0.04 for the combination of STI571 and doxorubicin.

In an *in vitro* C6 glioma test a C.I. of 0.3 ± 0.2 for the combination of STI571 and vinblastine was observed.

The same experimental protocol can be used to prove that STI571 induces synergistic interactions with other drugs as mentioned above.
The surprising beneficial effect of our new combinations, e.g. less side effects, synergistic therapeutic effect, can also be demonstrated in clinical studies as essentially described hereinafter in a manner known to the skilled person.

### Example 3:

The purpose of this study is to determine the effects of treatment of patients with osteolytic or osteoblastic bone metastases with the combination of Glivec + Zometa.

### STUDY OJECTIVES:

The primary objective of this study is to prove that there are synergistic drug interactions and no side-effects between Glivec and Zometa (pharmacokinetic, pharmacologic, toxic).
The secondary objective is to determine time to disease progression (skeletal, extra-skeletal) for patients treated with Glivec + Zometa.
Other objectives are to determine pain scores, analgesic scores, skeletal-related events, objective bone lesion responses, bone resorption markers, and overall survival for patients treated with Glivec + Zometa. It is also an objective to evaluate the efficacy of Glivec + Zometa in palliative treatment of painful bony metastases.

### DURATION OF STUDY:

Time permitted for patient enrollment: 12 months
Duration of an individual patient's participation: 12 months
Total duration of study: 24 months

### STUDY POPULATION: 15 Patients

Inclusion Criteria: Any cancer with at least one bone metastasis (lytic or blastic) on plain film (≥ 1 cm). Patients may be receiving anti-cancer therapy at the time of randomization. Ambulatory patients aged ≥18 years or the age of majority.
ECOG (Eastern Cooperative Oncology Group) performance status of 0, 1 or 2.
If the patient is of childbearing potential, a negative pregnancy test is required prior to treatment. Ability to comply with trial requirements.

### Interruption or Discontinuation of Treatment:

It is documented whether or not each patient completed the clinical study. If for any patient either study treatment or observations were discontinued the reason is recorded. Patients discontinuing trial treatment before 12 months are not replaced. Reasons that a patient may discontinue participation in a clinical study are considered to constitute one of the following:
Adverse event(s), abnormal laboratory value(s), abnormal test procedure result(s), unsatisfactory therapeutic effect, subject's condition no longer requires study treatment, protocol violation, subject withdrew consent, lost to follow-up, administrative problems, death.

### TREATMENT:

All patients receive the following: A) Zometa 4 mg as a 15 minute IV infusion every 3-4 weeks + Glivec 400, 600 or 800 mg P.O. daily (The 800 mg dose of Glivec is given as 400 mg bid.). On study day 1, eligible patients receive a 4 mg/15 min infusion of Zometa with attendant pharmacokinetic sampling. B) Glivec p.o. dosing is initiated on study day 3.
Pharmacokinetic sampling for steady-state imatinib concentrations takes place over an 8-hour period on any day during days 10-14, and on day 28 (coincides with second dose of Zometa). Study drug supplies (Zometa and Glivec) will be shipped to the pharmacist at each center. Study drugs will be supplied for the 12-month study period. Drugs are packaged in an open-label fashion.
Investigational therapy: Zometa is supplied in 4 mg lyophilized vials. Each 4 mg vial is to be reconstituted with 5 ml of sterile water. The appropriate volume of reconstituted Zometa is to be mixed with normal (0.9%) saline so that the total volume infused is 100 ml. Reconstituted Zometa is to be administered as a 15-minute i.v. infusion.

If reconstituted Zometa solutions cannot be used immediately, solutions must be refrigerated at temperatures between 36-46°F (2-8°C) and can be used for up to 24 hours.

Three patients are treated at 400, 600 or 800 mg per day with Glivec. If there are grade 3 or 4 toxicities in any patients, the number of patients at that dose of Glivec are expanded to 6 patients. The MTD (maximum tolerated dose) of Glivec is reached if 2/6 patients exhibit grade 3 or 4 toxicity. (See NCI/NIH Common Toxicity Criteria)
If an individual patient experiences grade 3 or 4 toxicity, treatment under this study with Zometa + Glivec is discontinued. That patient then receives treatment as directed by his/her physician.
An individual patient may continue to receive Zometa + Glivec for up to 12 months if there is disease progression in bone.

Standard Anti-Cancer Therapy: Patients must be receiving anti-cancer therapy at the time of randomization. Breast cancer patients receiving hormonal therapy must be on first-line or second-line hormonal therapy for metastatic disease. Breast cancer patients must not be receiving greater than second-line hormonal therapy for metastatic disease at the time of randomization, unless being given in combination with chemotherapy. Changes to the initial anti-neoplastic regimen during the study are at the discretion of the treating physician. Patients in both disease groups may discontinue all specific anti-neoplastic therapies at any time during the trial. Patients who are no longer receiving anti-neoplastic therapies must continue to receive study drug infusions at 3-4 weekly intervals until the stipulated 12-months of therapy is completed.

Blood Chemistry: During ongoing monitoring of the phase III trials with Zometa® the safety monitoring boards noted a possibly higher frequency of increases in serum creatinine and AEs associated with the use of Zometa® and recommended that serum creatinine is measured prior to each dose of study drug. A 72-hour window for checking creatinine is allowed prior to the next dose. The local serum creatinine result is evaluated according to the following criteria:
If the patient's baseline serum creatinine was < 1.4 mg/dl at the time of study entry, an increase of 0.5 mg/dl or more requires the delaying of the dose of study drug until the patient's serum creatinine returns to no higher than 10% above the baseline value.

If the patient's baseline serum creatinine was ≥ 1.4 mg/dl, then any increase in the serum creatinine of 1.0 mg/dl or more requires that the study drug be delayed until the patients serum creatinine returns to no higher than 10% above the baseline value. Any doubling of the baseline serum creatinine value requires that the study drug be delayed until the patient's serum creatinine returns to no higher than 10% above the baseline value. Should the study drug need to be delayed, the patient's serum creatinine continues to be followed at intervals according to the Investigator's clinical judgment, but at least at the regularly scheduled study visits until full recovery (i.e., return to no higher than 10% above the baseline value). Other study procedures should be followed according to the protocol schedule even if study drug continues to be held.

If the patient experiences a Grade 3/4 hematological toxicity, defined as an ANC < 1 x 10⁹/L, or a platelet count < 50 x 10⁹/L, Glivec must be withheld until the toxicity has resolved to ≤ Grade 2. ANC (absolute neutrophil count) will take precedence over a WBC (white blood cell) count in determining the degree of neutropenia (e.g. doses should not be interrupted for a patient with a WBC < 2.0 x 10⁹ /L but ANC > 1 x 10⁹/L). If the toxicity resolves to Grade 1 within two weeks, Glivec treatment may be resumed at the same dose. If the Grade 3/4 toxicity recurs or persists for longer than two weeks, Glivec must be withheld and then recommended at the daily dose reduced by 200 mg (e.g. from 800 mg/day to 600 mg/day) once toxicity has resolved to ≤ Grade 1. If the Grade 3/4 toxicity recurs again, Glivec must be stopped. No dose reductions will be performed again for grade 1-4 anemia. If the patient develops anemia, s/he may be transfused at the discretion of the investigator.
The optimal dose of Glivec in an as-yet undefined spectrum of diseases is unknown, however, the possibility of a clinically relevant dose-response may be of importance. Therefore, it is possible that higher doses of Glivec might induce responses even if a lower dose fails. If vomiting occurs, no additional trial medication should be taken that day in an effort to replace the material that has been vomited.
If for any reason, interruption of treatment with Glivec is equal or longer than 14 days, than the patient should be discontinued.

Skeletal-Related Events: SREs are defined as pathologic bone fractures, spinal cord compression, surgery to bone and radiation therapy to bone.
Pathologic fractures are defined as bone fractures which occur spontaneously or which result from trivial trauma. A new vertebral compression fracture is defined as a decrease in total vertebral height, or anterior vertebral height, or posterior vertebral height of ≥ 25% from baseline (Visit 1). An old (pre-existing) vertebral compression fracture may be present at Visit 1. At visit 1, a pre-existing vertebral compression fracture is defined as a decrease in total, or anterior or posterior vertebral height of ≥ 25% as compared to a previous spinal film or as compared to an adjacent vertebrae. A further reduction in the total, or anterior, or posterior vertebral height of an old vertebral fracture by ≥ 25% during the study is to be classified as a new vertebral compression fracture. Each pathologic fracture (vertebral and non-vertebral, including rib fractures) is to be documented by a plain X-ray film during the study and is to be counted separately.

Overall Progression of Disease: Overall progression of disease is determined every 3 months by the investigator using the tumor response criteria outlined in a form A (for multiple myeloma patients) and B (for breast cancer patients). The assessment of tumor progression must take into account the radiologist's assessment of serial bone radiographic studies and appropriate serial radiographic studies of non-skeletal tumor sites, if present. ECOG Performance Status: ECOG performance status is completed at least every 3 months.
Quality of Life (QOL): QOL is assessed by using a FACT-G (Functional Assessment of Cancer Therapy General) questionnaire every 3 months. The quality of life questionnaires is completed by the patient upon arrival at the clinic and before the patient has either been interviewed by the physician or received study medication.
Analgesic Scores: Analgesic use is scored every 3 months according to the analgesic score.
Pain Score: The Pain Score is assessed every 3 months according to the brief Pain Inventory (BPI) short form. The BPI can produce three pain scores: worst pain, a composite pain score and a pain interference score. The composite pain score is used in this study.
Markers of Bone Resorption: The following parameters are be measured by a central laboratory:
- Serum PTH (parathyroid hormone) (Visits 1, 6, 10, 14 and 19). Measurement of PTH using an immuno-chemiluminescent assay (ICMA-PTH) is performed. Draw a sample (5ml) in a 5.0 ml red-top or SST (serum separator) tube and centrifuge. Immediately after centrifugation, remove serum (2 ml) and place in plastic transport vial and freeze. Send the frozen serum in plastic vials on dry ice to the central laboratory. The data remain blinded to sites.
- BAP (every 3 months). Venous blood will be drawn for BAP (bone alkaline phosphatase). The data remain blinded to the study monitors and investigator sites.
- Urine chemistry (every 3months). After rising in the morning, the initial urine sample passed by the patient is discarded. The subsequent 'second void' urine is collected. The patient need not remain fasting but to reduce diurnal variation other sample is collected in mid-morning whenever possible. Urine must be mixed thoroughly before taking aliquots for creatinine, pyridinoline, deoxypridinoline and N-telopeptide. The data remain blinded to the study monitors and investigator sites.
Safety Assessments: Safety assessments consist of monitoring and recording all adverse event and serious adverse events, the regular monitoring of hematology, blood chemistry and urine values, and the performance of physical examinations. In addition the time to discontinuation of study drug is evaluated and survival will be assessed.
Drug levels and pharmacokinetic assessments: A blood sample (5mL) for the determination of serum concentration of alpha-1 acid glycoprotein is obtained at visit 1.
Sequential blood samples (5 mL) or the determination of plasma concentrations of zoledronic acid are obtained at visit 2 as follows; 0h (pre start infusion), 15 min (within 30 seconds post end infusion), 45 min, 1.5h, 3h, 5h, 8h.
Sequential bloods samples (5 mL, 7.5 mL for 0h sample) for the determination of plasma concentrations of imatinib (imatinib and zoledronic acid in 0-h sample) are obtained at visit 2A (any time between days 10-14 post first dose Zometa) as follows: 0h (pre-oral dose), 1.5 h, 3h, 5h, 8h.
Sequential blood samples (7.5 mL) for the determination of plasma concentrations of imatinib and zoledronic acid are obtained at visit 3 as follows: 0h (pre-oral Glivec and i.v. Zometa), 15 min, 45 min, 1.5h, 3h, 5h, 8h.
Sequential blood samples (7.5 mL) for the determination of plasma concentrations of imatinib and zoledronic acid are obtained at visit 6 as follows: 0h (pre-oral Glivec and i.v. Zometa), 15 min, 45 min, 1.5h, 3h, 5h, 8h.
These samples are stored at -20C and shipped to a laboratory to measure Glivec (imatinib) and Zometa (zoledronic acid) levels.

By this study it is shown that the toxicity profile of the Glivec/Zometa combination is superior to that of other combinations. Furthermore, the Glivec/Zometa combination shows a synergistic therapeutic effect, higher efficacy, efficacy in palliative treatment of painful bony metastases, and other beneficial effects as mentioned in the above description.

### Example 4:

### PROTOCOL SUMMARY

Study Objectives: To prove the safety and efficacy of Glivec (imatinib mesylate, ST1571) and Zometa (zoledronic acid) in the palliative treatment of painful bony metastases due to androgen-independent prostate cancer. Prove that the Glivec/Zometa combination shows a synergistic therapeutic effect, higher efficacy, improved safety and other beneficial effects as mentioned in the above description.
Patient Inclusion Criteria: Patients with histologically confirmed prostate cancer who have progressed on standard hormonal management (including antiandrogen withdrawal) and have symptomatic bone metastases are eligible. Eligible patients continue primary hormonal therapy, have a PSA (prostate specific antigen) of ≥ 5 ng/ml, an ECOG performance status ≤ 3, and adequate hematologic, hepatic and renal function.
Patient Exclusion Criteria: Patients with serious medical illness, active second malignancy other than non-melanoma skin cancers will not be eligible. Patients who have received radiotherapy within 30 days, bisphosphonates within 6 months, radiopharmaceuticals within 60 days, or investigational therapy within 30 days will be excluded from the study.

Treatment Schedule: Zometa 4 mg i.v. every 28 days and Glivec 600 mg po q.d.

Primary Objective: Pain response defined as a 2-point reduction on the 6 point present pain intensity scale of the mMcGill-Melzack Pain Questionnaire (Tannock I. et al.. Treatment of metastatic prostatic cancer with low-dose prednisone: evaluation of pain and quality of life as pragmatic indices of response. J Clin Oncol. 1989;7(5):590-7; Melzack R. The McGill Pain Questionnaire: major properties and scoring methods. Pain. 1975;1:277-299) maintained on 2 consecutive evaluations at least 4 weeks apart without an increase in analgesic consumption.

Secondary Objectives: 50% decrease in analgesic medication use without an increase in pain maintained on 2 consecutive evaluations at least 4 weeks apart.
PSA (prostate specific antigen) response defined as a 50% decrease in PSA maintained on 2 consecutive evaluations at least 4 weeks apart.

Impact of therapy on markers of bone turnover, specifically: serum bone specific alkaline phosphatase, serum osteocalcin, and urine N-telopeptides prior to treatment, and after 2, 4, 8, and 12 weeks of treatment.
Time to progression measured by PSA and pain endpoints.
Impact on quality of life measured by the EORTC core questionnaire QLQ-C30 (Aaranson NK, et al. The European Organization for Research and Treatment of Cancer QLQ-C30: a quality-of-life instrument for use in international clinical trials in oncology. Journal of the National Cancer Institute. 1993;85(5):365-376; Osoba D et al. Psychometric properties and responsiveness of the EORTC Quality of Life Questionnaire (QLQ-C30) in patients with breast, ovarian, and lung cancer. Quality of Life Research. 1994;3(353-364)) and a module specific for prostate cancer designed according to EORTC guidelines. Toxicity of the GLIVEC/Zometa combination.

### Example 5:

OBJECTIVES: Determine the doses of Glivec given in combination with letrozole, that are associated with steady-state concentrations that are similar to the concentrations achieved with glivec 600 mg/daily and 800 mg/d, given as a single agent. These doses will be the basis for further clinical studies.
Prove that the glivec/letrozole combination shows a synergistic therapeutic effect, higher efficacy, improved safety and other beneficial effects as mentioned in the above description.
Determine the safety with co-administration of letrozole and glivec.
Describe responses to combination therapy as assessed by clinical measurement or serial radiographic studies and evaluate progression-free survival for patients at each dose level. To measure the c-kit and PDGF-receptor expression level by immunohistochemistry on available tumor blocks and correlate with clinical response.
To determine pharmacokinetics for glivec and letrozole and relate to the function of CYP3A4 as assessed by the erythromycin breath test.

### STUDY DESIGN:

The study is an open-label, phase I study to assess the safety and pharmacokinetic profiles of letrozole and glivec when given in combination. Ten patients are enrolled at each dose level. The dose of letrozole is fixed at 2.5 mg/d orally. Patients receive letrozole alone for 2 weeks. On day 15 patients begin glivec and continue letrozole. The first dose level of glivec is 200 mg/day orally. If 10 patients are accured to the first level, without defining a maximum tolerable dose, the second dose level is 400mg/d. If 10 patients are accrued at 400mg/d, without defining a maximum tolerable dose, the third dose level is 600mg/d. If 10 patients are accrued at 600 mg/d, without defining a maximum tolerable dose, the fourth dose level is 800 mg/d. If 10 patients are accrued at 800mg/d, without defining a maximum tolerable dose, the fifth dose level is 1000mg. Patients continue on the study until there is evidence of disease progression, they have experimented unacceptable toxicity, or they elect to withdraw from the study.

### PATIENT ELIGIBILITY:

Inclusion criteria: Patients have histologically-confirmed, estrogen receptor positive (greater than 10% nuclear staining) metastatic adenocarcinoma of the breast that has progressed despite tamoxifen therapy. Progression on tamoxifen includes patients who initially responded to tamoxifen (including stable disease), patients who were primarily refractory to tamoxifen, and patients who could not tolerate tamoxifen due to side effects.

Patients have the following baseline laboratory values:
Absolute Granulocyte Count ≥ 1,500/mm³
Platelet Count ≥ 100,000/mm³
White Blood Count ≥ 3,000/mm³
Serum Creatinine Clearance (CrCl) ≥ 40ml/min
(CrCl= Wt (kg) x (140-age)*/72 x Cr. level, *female x 0.85)
Total Bilirubin ≤ 2.0mg/dl
AST≤ ALT 2.5xULN or 1.5xULN
Patients must be at least 50 years of age, have an ECOG performance status of 0, 1, or 2, be postmenopausal (greater than one year form last menstrual period, or FSH less than or equal to 35 IU/ml), not as a consequence of chemotherapy. Patients are able to give informed consent and not be required to have either measurable or evaluable disease. Patients with metastatic disease to bone only as detected by bone scan are included as evaluable disease.
Exclusion Criteria: Patients with lymphangistic spread of cancer to the lung or histoy of carcinomatous meningitis. Patients with active infection or evidence of a significant medical problem, which in the opinion of the principal investigator makes the patient, a poor study candidate, are not eligible for participating in this study.

### TREATMENT PLAN:

Letrozole: All patients begin letrozole 2.5 mg orally daily on day 1 and continue until the end of the study. Compliance with study medication is documented with a diary, which the patients keep and which is reviewed monthly. Patients are asked to bring pill bottles of study medications to each of their appointments.
Glivec: All patients begin glivec at one of five dose levels on day 15. The initial dose level is 200 mg orally daily. The second dose level is 400 mg orally daily. The third dose level is 600 mg orally daily. The fourth dose level is 800 mg orally daily. The fifth dose level is 1000 mg orally daily. All patients continue on glivec until the end of the study. Compliance with study medication is documented with a diary, which the patients keep and which is reviewed monthly. Patients are asked to bring pill bottles of study medications to each of their appointments.

Pharmacokinetic sampling: All patients have blood drawn for pharmocokinetic studies prior to the daily dose of drug and one hour after the daily dose on day 1 and 15. A single sample is drawn before the daily dose on day 22, 28 and 35. Blood is collected in 5ml green topped tubes. They are centrifuged at 2500 rpms for 3 minutes at room temperature (7°C). Serum is transferred to labeled, polypropylene tubes and frozen at -70°C. The samples are transported on ice to the laboratory where they remain frozen at -70°C until assays are performed to determine the serum concentration of glivec and letrozole using previously described methods (Buchdunger et al. Inhibition of the abl protein-tyrosine kinase in vitro and in vivo; Cancer Res 1996 Jan 1;56(1):100-4.
Erythromycin breath test are performed once at baseline.

Tumor blocks are obtained, when possible, on all patients. These include core needle biopsy, excisional biopsy and surgically resected specimens from either the primary or metastatic tumor.
Phase II dose is defined by the dose of glivec, in combination with letrozole, that achieves a steady-state concentration of glivec of 1.44 mcg/ml, with acceptable standard deviation of 0.40 mcg/ml [acceptable range 1.04-1.84 mcg/ml). The steady-state concentration is determined as the mean of the trough serum levels of glivec measured 24 hours after a dose of drug on days 22, 28 and 35.

Maximum Tolerated Dose: The maximum tolerated dose (MTD), is defined as follows. Dose limiting toxicities (DLTs), attributed to study drug(s) treatment, are defined as toxicities related to drug treatment requiring discontinuation of the study drug(s). It includes at least one of the followings: 1) grade 4 neutropenia, absolute neutrophil count (ANC) less than 500/mm³ lasting greater than five days; 2) grade 3 or 4, absolute neutrophil count (ANC) less than 1000/mm³ with fever greater than 101 °F; 3) grade 4 thrombocytopenia, platelet count less than 10,000/mm³; 4) nonhematologic toxicity ≥ CTC grade 3. The maximum tolerated dose (MTD) are defined as one dose level below the dose that induced dose-limiting toxicity in 4 or more patients at a dose level. Thus, the occurrence of DLT in four or more patients at a given dose level results in termination of the study. The MTD are defined as the dose level below the one with four out of ten patients experiencing DLTs, or fewer if, in the opinion of the principal investigator, unacceptable toxicity prohibits enrolling ten patients to a dose level.

### STUDY PARAMETERS:

Minimum laboratory values required on day 1 and 15:
   Serum Creatinine < 1.5 mg/dl
   Platelet Count > 100,000/mm3
   Absolute Granulocyte Count >_ 1,500/mm3
   Total Bilirubin _< 2.0 mg/dl
   AST/ALT _< 2.5 x ULN or 1.5 ULN
Clinical and Laboratory Assessments:
   Performance Status, Toxicity Notation, CBC with *differential,* BUN/creatinine, AST, ALT,alkaline phosphatase, bilirubin, total protein, albumin, sodium, potassium, blood glucose, calcium, phosphate, Tumor measurement, EKG, Erythromycin breath test.
   Tumor blocks: Tumor blocks are obtained on all patients, when possible. These include core needle biopsy, excisional biopsy and surgically resected specimens from either the primary or metastatic tumor. Immunohistochemistry are performed to evaluate for c-kit and PDGF-receptor overexpression with the validated technique currently in use in the Pathology Department at the Hospital of the University of Pennsylvania.

### EVALUATION CRITERIA:

Adverse Experiences: Patients are monitored for adverse experiences to include laboratory tests, physical finding, and patient reporting. These experiences are graded according to Common Toxicity Criteria scale. Serious Adverse Drug Reactions are defined as 1) previous unknown toxicities; 2) life-threatening of fatal toxicities regardless of whether or not previously unknown. We shall submit reports if there is a suspicion of drug effect. In addition to these serious adverse drug reactions, the IRB will also be notified of adverse events that 1) require or prolong hospitalization, 2) is permanently disabling, or 3) any overdose regardless of relationship to study drugs.

Evaluation for Response: RECIST (Response Evaluation Criteria in Solid Tumors) criteria for response (see World Health Organization): All measurable lesions up to a maximum of 10 lesions representative of all involved organs are identified as target lesions and are recorded and measured at baseline. Target lesions are selected on the basis of their size (lesions with the longest *diameter)* and their suitability for accurate repetitive measurements (either by imaging techniques or clinically). A sum of the longest diameter (LD) for all target lesions is calculated and reported as the baseline sum LD. The baseline sum LD is used as reference to further characterize the objective tumor response of the measurable dimension of *the* disease. All other lesions (or sites of disease) should be identified as non-target lesions and should also be recorded at baseline. Measurements are not required and these lesions should be followed as "present" or "absent". Target lesions are evaluated as follows:
Complete response (CR): Disappearance of all target lesions
Partial Response (PR): At least a 30% decrease in the sum of LD of target lesions taking as reference the baseline sum LD.
Progression (PD): At least a 20% increase in the sum of LD of target lesions taking as references the smallest sum LD recorded since the treatment started or the appearance of one or more new lesions
Stable Disease (SD): Neither sufficient shrinkage to qualify for PR nor sufficient increase to qualify for PD taking as references the smallest sum LD since the treatment started.
Patients who exhibit a partial response or disease stability after two cycles of therapy have the option to continue therapy with continued response evaluation after alternate cycles.
Responding patients continue therapy until disease progression, toxicity prohibits further dosing, or patient voluntarily withdraws.
Patients with measurable or evaluable disease are assessed at baseline, every three months during treatment, and at study termination to obtain any preliminary response data. The indicator tumor can be a primary or metastatic lesion found on diagnostic imaging. The method of measurement used at baseline is used to follow patients for response throughout the study. For patients with bone-only disease, bone scan will be the diagnostic test used to assess response. All patients are evaluated with CT scans regardless of location of the indicator tumor.
All responding Patients (CR/PR) have their response confirmed 4-6 weeks after the first documentation of response.

Correlation with immunohistochemistry: For the purposed of correlating clinical response with immunohistochemical assessment of c-kit and PDGF receptor overexpression, patients will be classified as having no clinical response or clinical response. Progressive disease is classified as non-responder. Clinical response includes CR, PR, or stable disease for at least six months; c-kit and PDGF receptor overexpression are scored by immunohistochemistry as 0, 1+, 2+, or 3+.

Safety Analyses: All patients are evaluated for safety and toxicity. A maximum of 10 patients are accrued to each dose level. If four patients at any dose level experience DLTs, then the MTD is defined as the next lower dose level. Safety analysis include the summaries of CTC grades, the dose adjustments, adverse event rates, laboratory changes, cumulative toxicity, DLT, time to and duration of neutropenia and thrombocytopenia at each dose level.

Efficacy Analyses: Clinical antitumor activity observed will be documented. Progression-free survival is calculated, for each patient, as the time from baseline to the earliest evidence of disease progression as assessed by tumor measurement. Tumor measurement can be made by physical examination, in the case of cutaneous or subcutaneous lesions. Otherwise, tumor measurement is made by appropiate radiographic study (e.g. CT scan, MRI or bone scan). This study is not powered to detect statistically significant differences in progression-free survival between treatment groups or compared to historical controls. This data is used for descriptive purposes and to generate further hypotheses. Kaplan-Meier estimates of progression-free survival are calculated with 95% confidence intervals.

Available tumor blocks are scored for overexpression of c-kit and PDGF receptor by imrnunohistochemistry. The scoring system for each assay is 0, 1+, 2+, or 3+. Patients' clinical response will be scored as no response (progressive disease) and positive response (stable disease for at least 6 months, minor response, partial response and complete response), Cross tabulations of clinical response (yes/no) against the ordinal measures of immunohistochemistry will be produced, primarily for hypothesis generation, however, on a preliminary basis, tests of trend is used to assess the associations of interest. These tests are performed separately for each dose, and overall across all doses.
By this study it is shown that the toxicity profile of the Glivec/letrozole combination is superior to that of other combinations containing no glivec. Furthermore, the Glivec/letrozole combination shows a synergistic therapeutic effect, higher efficacy and other beneficial effects as mentioned in the above description.

## Claims

1. Method of treating a warm-blooded animal having a proliferative disease or acute or chronic transplant rejection comprising administering to the animal a combination which comprises (a) N-{5-[4-(4-methyl-piperazino-methyl)-benzoylamido]-2-methylphenyl}-4-(3-pyridyl)-2-pyrimidine-amine and (b) a chemotherapeutic agent selected from anti-neoplastic agents and agents effective in treating acute or chronic transplant rejection, in a quantity which is jointly therapeutically effective against a proliferative disease or acute or chronic transplant rejection and in which the compounds can also be present in the form of their pharmaceutically acceptable salts or any hydrate thereof.

2. Method according to claim 1 wherein the chemotherapeutic agent is an antineoplastic agent selected from aromatase inhibitors, antiestrogens, topoisomerase I inhibitors, topoisomerase II inhibitors, microtubule active agents, alkylating agents, antineoplastic antimetabolites, platin compounds, compounds decreasing the protein kinase activity and further anti-angiogenic compounds, gonadorelin agonists, anti-androgens, bisphosphonates and trastuzumab.

3. Method according to claim 1 wherein the chemotherapeutic agent is selected from paclitaxel, doxorubicin, docetaxel, letrozole or zoledronic acid.

4. A combination which comprises (a) N-{5-[4-(4-methyl-piperazino-methyl)-benzoylamido]-2-methylphenyl}-4-(3-pyridyl)-2-pyrimidine-amine and (b) a chemotherapeutic agent selected from antineoplastic agents and agents effective in treating acute or chronic transplant rejection, wherein the active ingredients are present in each case in free form or in the form of a pharmaceutically acceptable salt or any hydrate thereof, and optionally at least one pharmaceutically acceptable carrier; for simultaneous, separate or sequential use.

5. Combination according to claim 4 wherein the compound (a) is used in the form of its monomethanesulfonate salt.

6. Combination according to claim 4 or 5 which is a combined preparation or a pharmaceutical composition.

7. Combination according to any one of claims 4 to 6 wherein the chemotherapeutic agent is selected from paclitaxel, doxorubicin, docetaxel, letrozole or zoledronic acid.

8. A pharmaceutical composition comprising a quantity which is jointly therapeutically effective against a proliferative disease or acute or chronic transplant rejection of a combination according to any one of claims 4 to 7 and at least one pharmaceutically acceptable carrier.

9. Use of a combination according to any one of claims 4 to 7 for the delay of progression or treatment of a proliferative disease.

10. Use of a combination according to any one of claims 4 to 7 for the preparation of a medicament for the delay of progression or treatment of a proliferative disease.

11. Use of a combination according to any one of claims 4 to 7 for the delay of progression or treatment of acute or chronic transplant rejection.

12. Use of a combination according to any one of claims 4 to 7 for the preparation of a medicament for the delay of progression or treatment of acute or chronic transplant rejection.

13. Use of a combination according to any one of claims 3 to 6 wherein the chemotherapeutic agent is zoledronic acid, for the palliative treatment of painful bony metastases.

14. Use of a combination according to any one of claims 3 to 6 wherein the chemotherapeutic agent is zoledronic acid, for the preparation of a medicament for the palliative treatment of painful bony metastases.

15. A commercial package comprising (a) N-{5-[4-(4-methyl-piperazino-methyl)-benzoyl-amido]-2-methylphenyl}-4-(3-pyridyl)-2-pyrimidine-amine and (b) a chemotherapeutic agent selected from antineoplastic agents and agents effective in treating acute or chronic transplant rejection, wherein the active ingredients are present in each case in free form or in the form of a pharmaceutically acceptable salt or any hydrate thereof, together with instructions for simultaneous, separate or sequential use thereof in the delay of progression or treatment of a proliferative disease or acute or chronic transplant rejection.

16. A commercial package according to claim 13 wherein the chemotherapeutic agent is selected from paclitaxel, doxorubicin, docetaxel, letrozole or zoledronic acid.
